# EUROPÄISCHE PATENTANMELDUNG

(11) **EP 2 886 134 A1**
(43) Veröffentlichungstag der Anmeldung: **24.06.2015**
(21) Anmeldenummer: 13199010.3
(22) Anmeldetag: 20.12.2013
(51) Int. Cl.: A61L 27/18, A61L 27/50, A61L 27/56, A61L 27/58

(54) **Resorbierbares Implantat**

(71) Anmelder: nolax AG, 6203 Sempach Station (CH)
(72) Erfinder: Häfner, Stephan, 8057 Zürich (CH); Buser, Stephan, 6210 Sursee (CH)
(74) Vertreter: Hepp Wenger Ryffel AG

(57) **Zusammenfassung**

Die Erfindung betrifft ein Implantat (1) zur Anwendung in einem Knochenhohlraum (5), welches im Wesentlichen synthetisches Material umfasst, insbesondere aus einem oder mehreren Polyurethanen, herstellbar oder hergestellt ist. Dabei weist das Implantat (1) einen mehrteiligen Aufbau (2) auf, welcher einen insbesondere offenporigen Schaum (3) und eine insbesondere mikroporöse Membran (4) umfasst. Das Implantat (1) ist resorbierbar, weist
a. viskoelastische Eigenschaften, insbesondere mit einer Druckfestigkeit von 0,0005 bis 0,1 N/mm², und/oder
b. einen Quelldruck, bevorzugt einen Quelldruck von 0,0001 bis 0,05 N/mm², besonders bevorzugt einen Quelldruck von 0,0004 bis 0,02 N/mm², auf.

Das Implantat zeichnet sich durch einfache Handhabbarkeit, vollständiges Ausfüllen eines Knochenhohlraums, vollständige Resorption und Kosteneffizienz aus.

## Beschreibung

Die Erfindung betrifft ein Implantat zur Anwendung in einem Knochenhohlraum, insbesondere in einem Alveolarraum und ein System zur Bereitstellung gemäss dem Oberbegriff der unabhängigen Ansprüche.

Gewebedefekte stellen speziell im Kieferbereich eine häufige Behandlungsindikation dar. Sowohl physiologische als auch pathologische Prozesse können ursächlich sein. Unabhängig davon sind sie immer mit funktionellen Nachteilen verbunden. Knochengewebe ist im physiologischen Zustand immer von Weichgewebe bedeckt, deshalb handelt es sich bei Knochendefekten meist um kombinierte Hart- und Weichgewebedefekte. Ein Beispiel dafür ist ein leeres Zahnfach (Alveole) nach Zahnextraktion mit einem zur Mundhöhle freiliegenden Alveolarknochen infolge gingivaler Diskontinuität im Bereich der ehemaligen Zahnwurzel. Zahnwurzeln und ihr Desmodont besitzen eine stabilisierende Wirkung auf den sie verankernden Alveolar- und Kieferknochen. Infolge eines Zahnverlustes beginnt eine Resorption, wodurch sich die Kieferkammdimensionen im Bereich der ehemaligen Zahnwurzel in vertikaler und horizontaler Richtung reduzieren. Der Körper reduziert das Hart- und Weichgewebeangebot auf das Minimum. Speziell der Knochenverlust in vertikaler Richtung ist irreversibel und der Knochen ohne aufwendige Rekonstruktion mit Knochentransplantaten nicht wiederherzustellen. Pathologische Prozesse wie chronische Parodontitiden hingegen, bei welchen der Zahnhalteapparat über die cervicale Eintrittspforte am Saumepithel mit pathogenen Keimen infiziert ist, führen zu entzündlichen Knochenresorptionen. Mit fortschreitender Destruktion des Zahnhalteapparates verlieren die Zähne ihre Verankerung, lockern sich und fallen schliesslich aus. Andere Pathologien manifestieren sich auch durch zystische Veränderungen mit platzfordernder und gewebeverdrängender Charakteristik. Dabei werden mitunter grosse Defekthohlräume im Kieferknochen gebildet, welche die Kieferkontur und Stabilität beeinträchtigen. Nach einer chirurgischen Entfernung der Pathologie verbleiben unterschiedlich grosse Knochendefekte, welche oftmals von Weichgewebedefekten begleitet sind und sich durch eine minderwertige Defektheilung (*reparatio*) kennzeichnen. Zur Regeneration des ursprünglichen Zustandes (restitio ad integrum) werden geeignete Gewebeersatzmaterialien eingesetzt.

Guided Tissue Regeneration (GTR) ist eine bekannte Methode in der Zahnmedizin, um Gewebedefizite zu regenerieren. Die Herausforderung besteht einerseits im Wettlauf zwischen langsam wachsendem Hartgewebe (Knochen) und schnell wachsendem Weichgewebe (Bindegewebe und Mukosa). Dabei wird eine Leitstruktur im Sinne eines Platzhalters zur Volumensicherung der unterschiedlich schnell regenerierenden Geweben zur Verfügung gestellt. Knochendefekte werden ohne Platzhalter immer von Weichgewebe besiedelt, bevor der Knochen in der ursprünglichen oder beabsichtigten Form regenerieren kann. Ein durch Weichgewebe gefüllter Knochendefekt entspricht einer *reparatio,* welche eine *restitutio ad integrum* verhindert. Vernarbendes Weichgewebe weist die Eigenschaft auf zu kontrahieren und übt somit zusätzlich ungünstigen und volumenreduzierden Druck auf das Wundgebiet aus. Knochendefekte heilen über ein sich spontan bildendes Blutkoagulum aus, wobei Wundblut, welches bereits Knochenstammzellen und Wachstumsfaktoren enthält, zunächst in den Defekt einblutet. Über den Blutgerinnungsprozess bildet sich ein Fibringerüst und schliesslich ein stabiles Blutkoagulum. Dieses Blutkoagulum ist jedoch fragil, gegen mechanische Einflüsse nicht beständig und beispielsweise in einer Extraktiosaveole zur Mundhöhle ungeschützt. Durch Flüssigkeiten wie Speichel oder Getränken erfährt das Blutkoagulum eine Reduktion in seiner Dimension oder zerfällt komplett. Da die Knochenregeneration jedoch hauptsächlich im Bereich des Koagulums stattfindet, muss dieses in seinem Volumen stabilisiert und vor Infektionen geschützt werden.

Implantate zur Verwendung im Alveolarraum sind beschrieben. Die DE 10 2008 010 893 B3 zeigt ein Implantat aus zwei Teilkörpern, wobei dieses aus Kollagen und/oder aus aufbereitetem oder synthetischen Knochenmaterial gefertigt sein kann. Das Implantat wird beispielsweise nach einer Zahnextraktion in den Alveolarraum zur Guided Tissue Regeneration (GTR) eingeführt. Dabei ist das Implantat nicht in der Lage komplexe Knochenhohlräume, wie beispielsweise nach einer Extraktion eines mehrwurzeligen Zahnes (Prämolare) offen liegend, auszufüllen. Nachteilig bei der Verwendung von Implantaten aus Kollagen ist die mangelnde Volumenkonstanz und Formstabilität, da Kollagen bei Flüssigkeitsaufnahme eine galert-artige Konsistenz annimmt und zunehmend im Volumen kollabiert. Somit ist es nicht in der Lage über einen längeren Zeitraum (Wochen bis Monate) eine strukturell und mechanisch stabile Platzhalterfunktion zu übernehmen. Insbesondere werden Kollagene für Hartgeweberegenerationen schnell vom Empfängerorganismus resorbiert. Die tierische Herkunft der Kollagene birgt darüber hinaus immer das Risiko einer Infektions-Übertragung von Tier auf Mensch. Implantate, die aus aufbereiteten tierischen (z.B. mineralischer Anteil von Rinderknochen) oder synthetischen (z.B. Hydroxylapatite) Knochenmaterialien gefertigt sind, weisen eine langsame und unvollständige Resorption auf mit Rückständen von bis zu 10 % nach 120 Monaten.

Die EP 1 575 636 B1 beschreibt ein Implantat bestehend aus einem porösen Gerüst aus synthetischen miteinander verbundenen Körnern und einer impermeablen Membran. Die Herstellung des Implantats wird anhand einer Abformung der extrahierten Zahnwurzel ermöglicht. Dies erfordert eine Extraktion *in* toto, was in vielen Fällen nicht möglich ist. Zähne mit komplexer Wurzelanatomie müssen in kleine Einzelteile zerlegt werden, um extrahiert werden zu können. Folglich lässt sich eine solche Wurzel nicht mehr abformen und falls doch aber aufgrund der komplexen Geometrie nicht wie beabsichtigt in einem Stück in die Alveole reponieren. Die Kombination von langsam resorbierendem beta-Tricalciumphosphat Granulat mit pH-ansäuernder PLGA-Beschichtung ist zudem nachteilig für die Wundheilung und die Geweberegeneration. Die Herstellung eines solchen Implantats ist zudem zeit- und kostenintensiv.

Es ist daher Aufgabe der Erfindung, die Nachteile des Standes der Technik zu überwinden. Insbesondere soll ein Implantat zur Verwendung in einem Knochenhohlraum bereitgestellt werden, welches sich durch einfache Handhabbarkeit, vollständiges, form- und volumenstabiles Ausfüllen eines Knochenhohlraums, selbständige Fixierung im Knochenhohlraum, vollständige Resorption und Kosteneffizienz auszeichnet.

Diese Aufgaben werden durch die in den unabhängigen Ansprüchen definierten Merkmale gelöst.

Die Erfindung betrifft ein Implantat zur Anwendung in einem Knochenhohlraum, insbesondere in einem Alveolarraum, welches im Wesentlichen synthetisches Material umfasst, insbesondere aus einem oder mehreren Polyurethanen, herstellbar oder hergestellt ist. Hierbei weist das Implantat einen mehrteiligen Aufbau auf, umfassend einen insbesondere offenporigen Schaum und eine insbesondere mikroporöse Membran. Der Schaum weist geschlossene oder offene Poren auf, die insbesondere eine Aufnahme und eine Verteilung von Gewebeflüssigkeit und/oder Blut vermitteln. Zudem gewährleisten die geschlossenen oder offenen Poren ein gesteuertes Einwachsen von neu gebildetem Knochengewebe und einem entsprechenden Gefässsystem. Die Membran dient als Barriere zwischen sich regenerierendem Weich- und Hartgewebe und ist entsprechend undurchlässig für Zellen. Dies wird über die Dichte, insbesondere den Durchmesser von in der Membran liegenden Poren bewerkstelligt. Die Membran ist jedoch durchlässig für Flüssigkeiten und Dampf, insbesondere Wasserdampf und lässt Botenstoffe, insbesondere Wachstumshormone, diffundieren. Die Verbindung von Membran und Schaum ist insbesondere stoffschlüssig.

Unter Alveolarraum wird hier und im Folgenden das Zahnfach verstanden.

Zur Herstellung des Implantats kann die Membran auf den vorgelegten Schaum aufgetragen werden, beispielsweise gesprüht, geschäumt oder gerakelt werden. Ebenso kann der Schaum auf die vorgelegte Membran aufgetragen werden, beispielsweise geschäumt oder als ausreagierter Schaum aufgetragen werden. Selbstverständlich können die Membran und der Schaum jeweils separat vorgelegt werden und mittels einer Zwischenschicht, insbesondere einer Polymerschicht, stoffschlüssig verbunden werden. Die Herstellung des Implantats ist ebenfalls möglich, indem der Schaum und die Membran coextrudiert werden.

Das Implantat zeichnet sich dadurch aus, dass es resorbierbar ist, dass es viskoelastische Eigenschaften aufweist, insbesondere mit einer Druckfestigkeit von 0,0005 bis 0,1 N/mm², und/oder einen Quelldruck, bevorzugt einen Quelldruck von 0,0001 bis 0,05 N/mm², besonders bevorzugt einen Quelldruck von 0.0004 bis 0.02 N/mm², aufweist.

Das bedeutet, dass das resorbierbare Implantat viskoelastische Eigenschaften insbesondere mit einer Druckfestigkeit von 0,0005 bis 0,1 N/mm², jedoch keinen Quelldruck aufweist, oder einen Quelldruck, bevorzugt einen Quelldruck von 0,0001 bis 0,05 N/mm², besonders bevorzugt einen Quelldruck von 0,0004 bis 0,02 N/mm², jedoch eine geringe Druckfestigkeit von kleiner 0,0005 N/mm² aufweist, oder sowohl viskoelastische Eigenschaften insbesondere mit einer Druckfestigkeit von 0,0005 bis 0,1 N/mm², als auch einen Quelldruck, bevorzugt einen Quelldruck von 0,0001 N/mm² bis 0,05 N/mm², besonders bevorzugt einen Quelldruck von 0,0004 N/mm² bis 0,02 N/mm², aufweist.

Unter viskoelastischen Eigenschaften versteht sich definitionsgemäss das Verhalten eines Stoffes der sowohl elastisch, bevorzugt komprimierbar, insbesondere ein-/zusammendrückbar oder einrollbar, ist und nach einem Zusammendrücken oder Einrollen ein ursprüngliches Volumen und ursprüngliche Abmessungen wieder erlangt. Dies kann in einer definierten Erholungszeit, insbesondere einer Erholungszeit zwischen 2 und 120 sec und bei insbesondere trockenen Umgebungsbedingungen erfolgen. Das Implantat liegt dabei insbesondere in einem getrockneten Zustand vor. Mittels der Zusammensetzung des Implantats kann Einfluss auf die Erholungszeit genommen werden. Nach einem applizierten Druck schwillt demnach das Implantat zu seiner ursprünglichen Dimension zurück. Unter einem Quelldruck versteht sich definitionsgemäss ein Druck, der sich aufbaut, wenn eine durch eine Wasseraufnahme ausgelöste Volumenzunahme (Quellung) eines Mediums behindert wird, beispielsweise durch eine Auflast. Erfindungsgemäss entwickelt das Implantat bei feuchten Umgebungsbedingungen einen Quelldruck, bevorzugt einen Quelldruck von 0,0001 bis 0,05 N/mm², besonders bevorzugt einen Quelldruck von 0,0004 bis 0,02 N/mm², vermittelt durch eine Aufnahme von Feuchtigkeit, insbesondere von Gewebeflüssigkeit und/oder Wundblut. Auf diese Weise löst das erfindungsgemässe Implantat die vorgenannten Aufgaben.

Die viskoelastischen Eigenschaften des Implantats können bei nassen Umgebungsbedingen eine Druckfestigkeit von 0,001 bis 0,1 N/mm² aufweisen. Unter feuchten Umgebungsbedingungen verstehen sich insbesondere Bedingungen, die sich in einer Mundhöhle, insbesondere in einer Alveole vorfinden.

In bevorzugten Ausführungsformen hat der Schaum definierte Breiten und definierte Höhen, vorzugsweise im Bereich von 0,5 bis 30 mm. Die Membran kann insbesondere eine Dicke von 0,01 bis 1,5 mm aufweisen.

Das Implantat kann derart ausgestaltet sein, dass die Membran den Schaum an einem in bestimmungsgemässer Anwendungssituation nach aussen weisenden Ende bedeckt. Dabei sind die Seiten und/oder das gegenüberliegende Ende nicht mit der Membran bedeckt. Die nicht von der Membran bedeckten Seiten sind dabei offenporig und ermöglichen das Einwachsen von Gewebe. Mit der Membran wird, insbesondere bei der Verwendung in einem Alveolarraum, eine gezielte Trennung von Weich- und Hartgewebe, insbesondere des Zahnfleischs und des Kieferknochens, erreicht.

Ein Einwachsen von Gewebe kann durch offene Poren an Membranfreien Seiten gewährleistet sein.

Das Implantat kann steril sein und/oder steril verpackt sein. Auf diese Weise wird eine Kontamination mit Pathogenen, insbesondere Mikroorganismen oder Entzündungserregern, des oder der zu generierenden Geweben vermieden und eine Anwendung unter sterilen Bedingungen ermöglicht.

Der Schaum und/oder die Membran des Implantats können aus einem oder mehreren Polyurethanen herstellbar oder hergestellt sein. Die Zusammensetzung der einen oder der mehreren Polyurethane umfasst dabei
- mindestens eine Polyol-Komponente, welche mindest eine Verbindung mit mindestens zwei, vorzugsweise zwei, drei oder vier Hydroxidgruppen umfasst;
- mindestens eine Polyisocyanat-Komponente oder mindestens ein Polyurethan-Präpolymer, welche mindesten eine Verbindung mit mindestens zwei, vorzugsweise zwei, drei oder vier Isocynatgruppen umfassen; und
- einen Zusatz, welcher mindestens eine der folgenden Substanzen enthält: Polyvinylpyrrolidon, Polyvinylpolypyrrolidon, sulfonierte Copolyester, Polyvinylalkylether, ein Copolymer von Polyvinylpyrrolidon mit Vinylacetat, Vinylimidazol oder Vinylcaprolactam, ein Copolymer von Alkylvinylether mit einer Säure oder einem Anhydrit, insbesondere Maleinsäure oder Maleinanhydrit, sowie Polymere und Copolymere der Acrylsäure und deren Salze oder Mischungen davon.

Auf diese Weise können Implantate mit definierter Festigkeit, Resorptionszeit und anforderungsgemässen Porengrössen im Schaum und/oder in der Membran hergestellt werden.

Die Zusammensetzung des einen oder der mehreren Polyurethane des Schaums kann zusätzlich mindestens eine oder Kombinationen der im Folgenden erläuterten Komponenten umfassen:
- mindestens ein porenformendes Agens, welches vorzugsweise ein zweiwertiges Fettsäuresalz, welches speziell in Form eines Pulvers bereitgestellt ist, umfasst;
- einen Porenstabilisator, welcher vorzugsweise ein Türkischrotöl oder Polyethersiloxan ist;
- mindestens einen Katalysator;
- mindestens ein Polycaprolacton, vorzugsweise Poly(ε-caprolacton);
- mindestens ein Mono- oder Polysaccharid, vorzugsweise Stärke;
- ein Polymer oder Copolymer einer Acrylsäure und ihres Salzes.

Vorteilhafterweise sollte ein Schaum keine Biofilm begünstigende Polysaccharide enthalten, dennoch aber über eine Komponente verfügen, die die positiven physikochemischen Eigenschaften von Polysacchariden imitieren kann und zugleich die Adhäsion von eukaryotischen Zellen fördert.

Durch Beifügen von Zusatz, welcher mindestens eine der folgenden Substanzen enthält: Polyvinylpyrrolidon, Polyvinylpolypyrrolidon, sulfonierte Copolyester, Polyvinylalkylether, ein Copolymer von Polyvinylpyrrolidon mit Vinylacetat, Vinylimidazol oder Vinylcaprolactam, ein Copolymer von Alkylvinylether mit einer Säure oder einem Anhydrit, insbesondere Maleinsäure oder Maleinanhydrit, sowie Polymere und Copolymere der Acrylsäure und deren Salze oder Mischungen davon, und durch geeignete Wahl der Polyol-Komponente quillt der Schaum bei Kontakt mit Gewebeflüssigkeit, Wundexsudat und/oder Blut. Dadurch passt sich der Schaum und/oder die Membran den Konturen des Defekts an und dieser wird so vollständig gefüllt. Infolge der Quellung fixiert sich das Implantat selbständig im Knochendefekt und übt einen konstanten Druck auf Knochendefektflächen aus. Es wird angenommen, dass ein solcher Druck einen positiven und stabilisierenden Stimulus auf das Knochengewebe hat und die Knochenresorption während der Knochenregeneration reduzieren oder auch gänzlich verhindern kann. Das neu gebildete Knochengewebe kann direkt an den Schaum als artifizielle extrazelluläre Matrix anhaften und in den Schaum einwachsen.

Der Zusatz, insbesondere das Polyvinylpyrrolidon, wird in der vorliegenden Erfindung in einer geringen Konzentration dem Polyethylenglykol beigemischt, wobei es nach Ablaufen der Reaktion grösstenteils in Form von "Inseln" sowohl auf der Oberfläche als auch im Innern des Polyurethanschaums verteilt ist. Dadurch ist die Konzentration des Zusatzes, insbesondere des Polyvinylpyrrolidons, auf dem Schaum partiell erhöht. Dies führt dazu, dass es selbst bei Zugabe einer geringen Menge an Zusatz, insbesondere an Polyvinylpyrrolidon, in der Zusammensetzung zur Bildung von Bereichen kommt, in welchen die Konzentration für biologische Aktivität ausreicht.

Ein besonderer Vorteil bei Anwesenheit von Polyvinylpyrrolidon ist, dass Polyvinylpyrrolidon Proteine binden kann. Dadurch werden Wachstumsfaktoren an der Diffusion gehindert und freie Matrixmetalloproteasen (MMPs) gebunden, so dass die Konzentration dieser im Bereich der nachwachsenden Zellen absinkt. Dies führt in der Folge zu einer positiven Beeinflussung der Wundheilung beziehungsweise der Geweberegeneration.

Überraschend ist zudem, dass durch die Verwendung von Polyvinylpyrrolidon im Schaum und/oder der Membran die Adhäsion und Formierung eines bakteriellen Biofilms vermindert wird, es aber gleichzeitig zu einer Adhäsion von eukaryotischen Zellen kommt. Ohne dass die Erfindung auf einen Wirkmechanismus zu beschränken wäre, wird derzeit vermutet, dass für die Wundheilung sich zunächst eine provisorische extrazelluläre Matrix aus Proteinen bilden muss, die als Adhäsionsgrundlage für die Zellen dient. Dies wird vermutlich durch die Anwesenheit von Polyvinylpyrrolidon begünstigt.

Weitere Vorteile bei der Verwendung von Polyvinylpyrrolidon als Ersatzstoff für Polysaccharide im Schaum und/oder in der Membran sind die gute biologische Verträglichkeit und die toxikologische Unbedenklichkeit.

Polyvinylpyrrolidon ist beispielsweise erhältlich als Kollidon 17® PF.

Die Polyolkomponente enthält mindestens eine Verbindung mit mindestens zwei oder mehr Hydroxidgruppen. Vorzugsweise besteht die Polyolkomponente aus einem Gemisch von zwei oder mehr Verbindungen mit mindestens zwei oder mehr Hydroxidgruppen. Bevorzugte derartige Verbindungen sind dabei hydroxidterminierte Polyether wie z.B. α,ω-Dihydroxypoly(oxyethylene), α,ω-Dihydroxypoly(1,2-ethylenoxid), α,ω-Dihydroxypoly(1,2-propylenoxid), α,ω-Dihydroxypoly(1,3-trimethylenoxid), α,ω-Dihydroxypoly(1,4-tetramethylenoxid), α,ω-Dihydroxypoly(methylenoxy-1,2- ethylenoxid) und dergleichen sowie Copolymere davon, mit Molmassen vorzugsweise bis zu 15000 g/mol, hydroxyterminierte aliphatische Polycarbonate wie z.B. α,ω- Dihydroxypoly(ethylencarbonat), α,ω-Dihydroxypoly(1,2-propylencarbonat), α,ω- Dihydroxypoly(1,3-propylencarbonat), α,ω-Dihydroxypoly(tetramethylencarbonat), α,ω-Dihydroxypoly(hexamethylencarbonat) und dergleichen sowie Copolymere davon, jeweils mit Molmassen vorzugsweise bis zu 15'000 g/mol, Polyanhydride aus Dicarbonsäuren, wie z.B. Malonsäure, Bernsteinsäure, Glutarsäure und dergleichen sowie Copolymere daraus, jeweils mit Molmassen vorzugsweise bis zu 15'000 g/mol, niedermolekulare zwei oder mehrwertige Alkohole wie z.B. Glykol, Polyethylenglykol, 1,2- Propylenglykol, 1,3-Propylenglykol, Butandiol, Pentandiol, Hexandiol und längerkettige lineare oder verzweigte aliphatische Diole, Glycerin, Triethanolamin, Pentaerythrit, 2,2-Bis(hydroxymethyl)propanol und dergleichen, Hydroxidgruppenenthaltende Aminosäuredimere, -trimere oder -oligomere, z.B. aus Tyrosin und/oder Serin, sowie Zuckeralkohole wie Sorbit und andere Naturstoffe oder Naturstoffderivate mit mindestens zwei Hydroxidgruppen und dergleichen. Des Weiteren können Polyestertriole wie Rizinusöl und Türkischrotöl eingesetzt werden.

Bevorzugt werden als Polyol-Komponenten Polyestertriole zugesetzt, die eine höhere Verzweigungsdichte bei der Vernetzung des Polyurethanschaums hervorrufen. Besonders bevorzugt wird Rizinusöl eingesetzt.

Besonders bevorzugt werden in der Polyolkomponente Polyester eingesetzt, deren Endgruppen Hydroxidgruppen sind. Beispiele derartiger Verbindungen sind Polycaprolactondiol und Polycaprolactontriol (z.B. unter der Bezeichung Capa kommerziell von Solvay erhältlich). Weitere Beispiele sind α,ω-Dihydroxypoly(D,L-lactid), α,ω-Dihydroxypoly(D-lactid), α,ω-Dihydroxypoly(L-lactid), α,ω-Dihydroxypoly(glycolid), α,ω-Dihydroxypoly (hydroxybutyrat) und andere aliphatische Polyester sowie deren Copolymere einschliesslich segmentierter Blockcopolymere aus Polyether und Polyestersegmenten, wie sie z.B. aus der Umsetzung von hochmolekularen Polyestern mit hydroxidterminierten Poly (alkylenglykolen) erhalten werden können, sowie Mischungen aus derartigen Polyolen.

Besonders bevorzugt werden solche Verbindungen eingesetzt, die bioverträglich und vom Körper resorbierbar sind. Beispielsweise sind dies Poly(ε-Caprolacton) (PCL), Poly(ε-Caprolacton-Co-Glycolid-Co-DL-Lactid), verzweigte sowie unverzweigte Polyethylenglykole (PEG), PCL-b-PEG-b-PCL, (α,ω-Dihydroxy-Oligo(((R)-3-Hydroxybutyrat-Co-(R)-3-Hydroxyvalerat)-Block-Ethylenglykol).

Für die Herstellung des Schaums und/oder der Membran können Polyurethan-Präpolymere verwendet werden. Im Allgemeinen weisen die in der vorliegenden Erfindung verwendeten Präpolymere eine molare Masse zwischen 400 und 15'000, bevorzugt zwischen 400 und 10'000 und besonders bevorzugt zwischen 400 und 1'000 auf.

Neben der Verwendung eines Polyurethan-Präpolymers kann eine Polyisocyanatkomponente mit mindestens einer Verbindung mit mindestens zwei Isocyanatgruppen in der Zusammensetzung verwendet werden. Die Polyisocyanatkomponente enthält dabei vorzugsweise mindestens ein biokompatibles aliphatisches Polyisocyanat oder mindestens eine von einem biokompatiblen Polyamin abgeleitete Verbindung. Bevorzugte Verbindungen sind dabei unter den Folgenden ausgewählt: gegebenenfalls substituierte Alkylendiisocyanate mit 3 bis 12 Kohlenstoffatomen wie Hexamethylendiisocyanat, oder Lysindiisocyanat, gegebenenfalls substituerte Cycloalkylendiisocyanate mit 5 bis 15 Kohlenstoffatomen wie Cyclohexylendiisocyanat, gegebenenfalls substituerte Alkylcycloalkylendiisocyanate mit 6 bis 18 Kohlenstoffatomen wie Isophorondiisocyanat, gegebenenfalls substituierte aromatische Diisocyanate wie p-Phenylendiisocyanant, Toluyldiisocyanate (alle Isomere sowie deren Mischungen), 4,4'-Diphenylmethandiisocyanat, sowie Isomere, Trimere und höhere Oligomere dieser Diisocyanate, Uretdione aus diesen Isocyanaten, Cyanurate und Isocyanurate aus diesen Isocyanaten und dergleichen.

Besonders bevorzugt werden Hexamethylendiisocyanat, 1,6-Diisocyanatohexan (HDI), 1,4-Diisocyanatobutane (BDI), Isophorondiisocyanat (IPDI), Dicyclohexylmethandiisocyanat (H12MDI), Lysinmethylesterdiisocyanat (LDI) oder 4.4'-Diphenylmethan-diisocyanat (MDI) eingesetzt.

Als Porenöffner eignen sich vorzugsweise nicht toxische Substanzen, deren Konzentration auch nach Resorption in den Körper nicht toxisch sind, bestehend aus einem divalenten Metallsalz einer langkettigen Fettsäure mit 1 bis 22 Kohlenstoff Atomen. Bevorzugt werden Porenöffner eingesetzt, welche Calciumstearat enthalten. Die bevorzugte Konzentration an Porenöffner in der Zusammensetzung der vorliegenden Erfindung liegt zwischen 0.01 und 5 Gew.-%.

Die Zusammensetzung kann ferner Porenstabilisatoren enthalten, die vorzugsweise nicht toxisch sind und deren Konzentration auch nach deren vollständiger Resorption durch den Körper keine toxischen Werte erreichen. Vorzugsweise werden als Stabilisatoren nicht ionische und anionische Tenside eingesetzt. Beispielsweise können als Porenstabilisatoren ein Polyethersiloxan, ein Salz einer sulfonierten Fettsäure oder ein Salz einer Fettsäure eingesetzt werden. Bevorzugt wird ein Salz einer sulfonierten Fettsäure, besonders bevorzugt Türkischrotöl (sulfoniertes Rizinusöl) verwendet. Die Konzentration der eingesetzten Stablisiatoren liegt in der vorliegenden Erfindung bei 0,001 bis 3 Gew.-%.

Darüber hinaus kann die Zusammensetzung auch nicht toxische Katalysatoren enthalten, deren Konzentration auch nach Resorption in den Körper nicht toxisch ist und die die Reaktion zwischen Hydroxid- und Isocyanatgruppen katalysieren. Bevorzugt wird in der vorliegenden Erfindung ein organometallischer Katalysator und/oder ein tertiärer Amin-Katalysator verwendet. Besonders bevorzugt werden dabei Bismutkatalysatoren und/oder 2'2'-Dimorpholinyldiethylether verwendet. Die Menge an eingesetzten Katalysatoren beträgt insbesondere 0.01 bis 1 Gew.-%.

Im Weiteren können auch weitere Komponenten wie Emulgatoren und dergleichen in der Zusammensetzung enthalten sein.

Als Zusatz können ebenfalls Copolymere aus Vinylpyrrolidon mit Vinylacetat, Vinylimidazol oder Vinylcaprolactam verwendet werden (z.B. unter den Namen Luvitec VA64W, VA64, VPI55K72W und VPC55K65W von BASF erhältlich). Besonders bevorzugt wird ein Copolymer aus Vinylpyrrolidon und Vinylimidazol verwendet, da die Imidazolstruktur die Bindung an Proteine fördert.

Der Zusatz kann auch sulfonierte Copolyester (z.B. unter dem Namen Eastman AQ-Polymere von Eastman erhältlich), Polyvinylalkylether, bevorzugterweise Polyvinylmethylether (unter dem Namen Lutonal M40 100% von BASF erhältlich), oder Polyvinylpolypyrrolidon enthalten.

Weitere bevorzugte Substanzen, die im Zusatz enthalten sein können, sind Copolymere von Alkylvinylether, bevorzugt Methyl- oder Ethylvinylether, mit einer Säure oder einem Anhydrit, bevorzugt Maleinsäure- oder Anhydrit (z.B. unter dem Namen Gantrez AN 169 oder Gantrez S-Reihe bei ISP erhältlich). Weiter bevorzugt enthält der Zusatz Copolymere von Polyacrylat und Natrium-Polyacrylat (beispielsweise unter dem Namen Superabsorber T 5066 F bei Degussa erhältlich).

Die Zusammensetzung des einen oder der mehreren Poylurethane des Schaums und/oder der Membran kann mindestens eine Polyol-Komponente in Mengen von 1 bis 96 Gew.-% aufweist umfassen, vorzugsweise in Mengen von 30 bis 60 Gew.-%, besonders bevorzugt in Mengen von 35 bis 50 Gew.-%.

Der Schaum kann das mindestens eine Polyurethan-Präpolymer oder die mindestens eine Polyisocyanat-Komponente in Mengen von 4 bis 60 Gew.-%, vorzugsweise in Mengen von 40 bis 60 Gew.-%, besonders bevorzugt in Mengen von 45 bis 55 Gew.-%, aufweisen.

Der Zusatz kann in Mengen von 0,001 bis 10 Gew.-%, vorzugsweise zwischen 1 und 4 Gew.-%, in der Zusammensetzung des Schaums und/oder der Membran eingesetzt werden.

Als Zusatz wird bevorzugt Polyvinylpyrrolidon in der Zusammensetzung des einen oder der mehreren Polyurethane des Schaums und/oder der Membran eingesetzte. Hierbei kann das Polyvinylpyrrolidon aus Polyvinylpyrrolidon mit einem bestimmten mittleren Molekulargewicht oder einer Mischung von Polyvinylpyrrolidon mit verschiedenen mittleren molaren Massen bestehen. Bevorzugt wird Polyvinylpyrrolidon mit einem mittleren Molekulargewicht von 7'000 bis 1'000'000 g/mol oder eine Mischung davon als Dispersion eingesetzt. Besonders bevorzugt wird Polyvinylpyrrolidon mit einem mittleren Molekulargewicht von 40'000 bis 60'000 g/mol eingesetzt (z.B. unter dem Namen Luvitec K30 von BASF erhältlich).

Die mindestens eine Polyol-Komponente des einen oder der mehreren Polyurethane des Schaums und/oder der Membran kann mindestens ein Polyethylenglycol umfassen.

Die mindestens eine Polyol-Komponente des einen oder der mehreren Polyurethane des Schaums und/oder der Membran kann mindestens ein Polycaprolacton, vorzugsweise ein Poly(ε-caprolacton), aufweisen.

Die Resorptionsgeschwindigkeit des Schaums und/oder der Membran lässt sich durch geeignete Wahl des Polyethylenglykols, des Mischverhältnisses der Polyethylenglykole in einer Mischung aus Polyethylenglykolen mit verschiedenen mittleren Molekulargewichten, durch die eingesetzte Menge an Poly(ε-Caprolacton) wie auch dem Verhältnis der Menge des Poly(ε-Caprolacton) zur Menge des mindestens einen Polyethylenglykols, dem Zusatz und dem Wasseranteil, sowie dem Mischverhältnis zwischen der Isocyanat- und der Polyolkomponente einstellen. Zudem lässt sich dadurch auch die Weichheit des Schaums und/oder der Membran einstellen.

In einer besonders bevorzugten Ausführungsform der Erfindung wird als Polyol-Komponente eine Mischung mit mindestens einem Polyethylenglykol und zusätzlich einem Poly(ε-Caprolacton) verwendet. Das mindestens eine Polyethylenglykol kann dabei verzweigt oder unverzweigt sein und ein mittleres Molekulargewicht von 100 bis 1'5000 g/mol, bevorzugt von 300 bis 5'000 g/mol, aufweisen. Bevorzugt umfasst die Polyolkomponente eine Mischung aus Polyethylenglykolen mit verschiedenen mittleren Molekulargewichten. Das mindestens eine Poly(ε-Caprolacton) kann dabei verzweigt oder unverzweigt sein und eine mittlere molare Masse von 100 bis 1'5000, bevorzugt von 100 bis 1'000, aufweisen.

Der in der vorliegenden Erfindung beschriebene Schaum dient unter anderem als artifizielle extrazelluläre Matrix für einwachsende Zellen und ist deshalb vorzugsweise porös. Die Poren können untereinander verbunden sein (offenporige Struktur), insbesondere Kanäle bilden, aber auch gegeneinander abgetrennt (geschlossenporige Struktur). Besonders bevorzugt weist der Schaum eine offenporige Struktur auf. Der prozentuale Anteil offener Poren beträgt dabei mindestens 70%, besonders bevorzugt mindestens 90%. Im trockenen Zustand weisen die Poren einen durchschnittlichen grössten Durchmesser von 40 bis 800 µm auf, wodurch ein gesteuertes Einwachsen von Knochestrukturen ermöglicht wird. Da der der Erfindung zugrunde liegende Schaum bei Kontakt mit Flüssigkeiten, insbesondere Wundexsudat quellen kann, weisen die Poren des Schaums im nassen, d.h. mit Flüssigkeit, Wundexsudat oder bevorzugt Blut durchtränkten Zustand, einen durchschnittlichen grössten Durchmesser zwischen 50 und 1000 µm auf. Besonders bevorzugt beträgt der durchschnittliche grösste Durchmesser der Poren im nassen Zustand zwischen 100 und 850 µm.

Der Schaum kann eine oder mehrere biologisch aktive Verbindungen, vorzugsweise bereitgestellt in kristalliner Form, aufweisen. Der in der vorliegenden Erfindung beschriebene Schaum kann mit einer flüssigen biologisch aktiven Substanz oder einem flüssigem Wirkstoff ausgestattet werden, indem der Schaum zunächst mit einem flüssigen Wirkstoff getränkt und anschliessend getrocknet wird. Dadurch bleibt der Wirkstoff in kristalliner Form an den Porenwänden zurück.

Biologisch aktive Verbindungen können vorzugsweise auch an den eingesetzten Polymeren angebracht sein. Dadurch kann eine kontrollierte Freisetzung dieser Verbindungen im Rahmen der Degradation des Polymers stattfinden.

Des Weiteren kann die Oberfläche des in der vorliegenden Erfindung beschriebenen Schaums modifiziert werden und mit festen biologisch aktiven Substanzen ausgestattet werden. Ein mögliches Verfahren dazu ist die Festphasensynthese nach Merrifield. Weitere, dem Fachmann bekannte Verfahren können ebenfalls angewendet werden.

Biologisch aktive Substanzen können dabei Wirkstoffe, Moleküle oder Komponenten sein, die einen biologischen oder chemischen Effekt in einer Defektstelle auslösen. Biologisch aktive Substanzen können dabei synthetische Moleküle, Biomoleküle oder Gemische dieser sein und umfassen Enzyme, organische Katalysatoren, Ribozyme, Organometalle, Proteine, Glykoproteine, Peptide, Polyaminosäuren, Antikörper, Nukleinsäuren, Steroide, Antibiotika, antivirale und antifugale Substanzen, Analgetika, Immunsuppressiva, Cytokine, Kohlenhydrate, oleophobe Substanzen, Lipide, Komponenten der natürlichen extrazellulären Matrix, Pharmazeutika und Therapeutika. Bevorzugt werden Wirkstoffe, die den Wundheilungsprozess unterstützen wie Antiseptika, Antibiotika, Analgetika, Polysaccharide, Komponenten der natürlichen extrazellulären Matrix und Wachstumsfaktoren verwendet. Wachstumsfaktoren können dabei TGF-β (transforming growth factor β), IGF-I und -II (insulin-like growth factors 1 and 2), PDGF (plateled derived growth factor), EGF (epidermal growth factor), VEGF (vascular endothelial growth factor), KGF (keratinocyte growth factor), FGF-2 (fibroblast growth factor 2), HGF(hepatocyte growth factor), BMP's (bone morphogenic proteins) und weitere bekannte Faktoren umfassen. Besonders bevorzugt eingearbeitet wird die Kombination der Wachstumsfaktoren PDGF, VEGF, FGF-2 und BMPs, da diese die Angiogenese zusammen am besten fördern.

Zusätzlich kann der Schaum in der vorliegenden Erfindung mit einer Saccharidkomponente, wie Monosacchariden wie beispielsweise Dextrose, Mannose, Mannit, Dulcit, Glukose, Fruktose, Galaktose und dergleichen, Disaccharide wie beispielsweise Maltose, Laktose, Saccharose, Cellobiose und dergleichen; Oligo- und Polysaccharide wie beispielsweise Cellulose, Carboxymethylcellulose, Nanocellulose, reduzierte oxidierte Cellulose, Pektin, Amylopektin, Amylose, Chitin, Chitosan, Alginate und dergleichen sowie Mischungen davon versetzt werden. Besonders bevorzugt wird beim erfindungsgemässen Schaum Stärke eingesetzt, da es sich um ein kostengünstiges Polysaccharid handelt.

Der Schaum kann ein Muster aufweisen, welches das Schneiden des Schaums auf spezifische Abmessungen und Formen, wobei die Quelleigenschaften des Schaums berücksichtigt werden, ermöglicht.
Bevorzugt kann der Schaum auf eine Trägerfolie, insbesondere eine Polymerfolie appliziert sein, auf der ein Schnittmuster unter Berücksichtigung der Quelleigenschaften angegeben ist, was das Zuschneiden des Implantats auf eine bestimmte Grösse und/oder Form erleichtert.

In einer besonderen Ausführungsform kann der Schaum zur lokalen Applikation mit variabler Freisetzungskinetik eines Wirkstoffs verwendet werden, beispielsweise zur Stillung einer Blutung. Da der Schaum im Körper resorbiert wird, erfolgt eine kontinuierliche Abgabe des Wirkstoffs bis der Schaum vollständig abgebaut ist. Besonders geeignet ist die Verwendung des erfindungsgemässen Schaums oder der Membran des Implantats für eine kontinuierliche Abgabe eines Wirkstoffs oder einer Kombination von Wirkstoffen.

In einer weiteren bevorzugten Ausführungsform der Erfindung weist die Verpackung des Implantats eine Messtabelle auf, anhand derer unter Berücksichtigung der Quelleigenschaften des Schaums das Zuschneiden des Implantats auf bestimmte Grössen und/oder Formen erleichtert wird.

Der Schaum des Implantats kann herstellbar oder hergestellt sein
- mittels eines insbesondere kontinuierlichen Sprühauftragsverfahren, oder
- in einem Formwerkzeug geschäumt, oder
- aus einer Suspension gefriergetrocknet werden.

Bevorzugt wird der Schaum des Implantats in einem Formwerkzeug hergestellt. Das Reaktionsgemisch wird in die Form eingebracht. Die Komponenten werden dabei kurz zuvor in einer Mischkammer zusammengemischt. Zur Expansion des Schaums kann ein zugesetztes Treibmittel (Stickstoff, CO₂, gasförmiger Kohlenwasserstoff, thermisch aktivierbares Treibmittel etc.) oder das bei der Reaktion der Komponenten entstehende CO₂ verwendet werden. Besonders bevorzugt wird Wasser als Treibmittel benutzt, um eine Porenstruktur zu erhalten. Je nach der Menge des in der Zusammensetzung vorhandenen Wassers lässt sich zudem Durchmesser und Anzahl der Poren beeinflussen. Das bei der Reaktion entstehende CO₂ führt zur Porenbildung. Der Schaum kann so in einer Form geschäumt werden. Die so entstandenen Schaumblöcke werden anschliessend auf die benötigten Dicken, vorzugsweise zwischen 0.3 und 30 mm, besonders bevorzugt zwischen 0.5 und 25 mm, zurechtgeschnitten.

Erfindungsgemäss wird unter einer Membran eine planare, ununterbrochene drei-dimensionale Struktur verstanden. Die Membran weist dabei eine maximale Dicke von 1500 µm auf. Des Weiteren kann die Membran mikroporös im Sinne von durchgängig für Gase, Flüssigkeiten und Botenstoffe, jedoch unpassierbar für andere Substanzen, insbesondere Mikroorganismen, sein. Hierbei weist die Membran vorzugsweise Poren auf, welche ein durch die Membran Hindurchgelangen von Mikroorganismen mit einem Durchmesser zwischen 0,5 und 10 µm verhindern.

In einer bevorzugten Ausführungsform weist die Membran des Implantats eine Dicke von 10 bis 1'000 µm auf, vorzugsweise eine Dicke von 20 bis 500 µm, besonders bevorzugt eine Dicke von 50 bis 300 µm.

Die Membran kann das mindestens eine Polyurethan-Präpolymer oder die mindestens eine Polyisocyanat-Komponente in Mengen von 4 bis 60 Gew.-%, vorzugsweise in Mengen von 40 bis 60 Gew.-%, besonders bevorzugt in Mengen von 45 bis 55 Gew.-%, aufweisen.

Bevorzugt wird die Membran hergestellt durch
- Ausrakeln des Reaktionsgemischs in gewünschter Dicke auf einem Substrat;
- Auftrag über eine Breitschlitzdüse direkt auf die Schaumoberfläche oder auf ein Substrat; oder
- Sprühauftrag direkt auf die Schaumoberfläche oder auf ein Substrat.

Das Substrat kann beispielsweise ein silikonisiertes Papier oder eine Stahloberfläche sein. Auf diese Weise lassen sich besonders einfach die erfindungsgemässen stoffschlüssigen Schaum-Membran-Kombinationen mit den gewünschten Dimensionen herstellen. Ebenfalls möglich ist eine Walzenauftragung, bei der das Polymer mit Hilfe mindestens einer Walze auf das Substrat aufgetragen wird. Alternativ kann die Membran auch durch eine Extrusion hergestellt werden, bei der ein ausreagiertes Polymer zu einem Granulat verarbeitet, dieses anschliessend erhitzt und über hohen Druck durch eine Breitschlitzdüse direkt auf den Schaumkörper extrudiert wird.

Des Weiteren betrifft die Erfindung ein System im Sinne eines Kit-of-parts mit verschiedenen Implantaten. Die Implantate weisen hierbei unterschiedliche Abmessungen, vorzugsweise im Bereich von 0,5 bis 30 mm respektive von 0,125 bis 27'000 mm³, unterschiedliche Porengrössen des Schaums, insbesondere mit Porengrössen eines Durchmessers im Bereich zwischen 50 und 1000 µm im nassen Zustand, und/oder unterschiedliche Porengrössen und Abmessungen der Membran auf. Weiter umfasst das System Instruktionen zur Anwendung des Implantats in einem Knochenhohlraums, insbesondere in einem Alveolarraum. Bevorzugt kann das System Implantate mit unterschiedlichen Resorptionseigenschaften, insbesondere mit unterschiedlichen Resorptionsgeschwindigkeiten, aufweisen. Die Resorptionsgeschwindigkeit der einzelnen Implantate, insbesondere der Komponenten Schaum und/oder Membran der Implantate, lässt sich durch die bei der Herstellung eingesetzte Menge an Polycaprolacton und/oder Polyvinylpyrrolidon einstellen. Bevorzugt kann die Resorptionsgeschwindigkeit auch durch die eingesetzte Menge an Wasser, durch Auswahl der Polyolkomponente sowie das Mischverhältnis zwischen Isocyanat- und der Polyolkomponente beeinflusst werden. Hierbei sind die Implantate insbesondere einzeln steril verpackt, was eine Verwendung des Implantats unter sterilen Bedingungen ermöglicht. Durch den zur Verfügung gestellten Satz an Implantaten mit verschiedenen Eigenschaften und Abmessungen kann bei der Anwendung in einer Defektstelle jeweils ein Implantat mit der für die Defektstelle optimalen Resorptionsgeschwindigkeit und Abmessungen ausgewählt werden.

Das erfindungsgemässe Implantat wird anhand von Abbildungen im Folgenden näher erläutert.

Es zeigen:
- Figur 1: Eine schematische Seitenansicht eines erfindungsgemässen Implantats (A) und seines mehrteiligen Aufbaus (B);
- Figur 2: Schematische Seitenansichten erfindungsgemässer Ausführungsformen (A-H);
- Figur 3: Schematische Seitenansicht eines erfindungsgemässen Implantats in einem Knochenhohlraum (A-B).

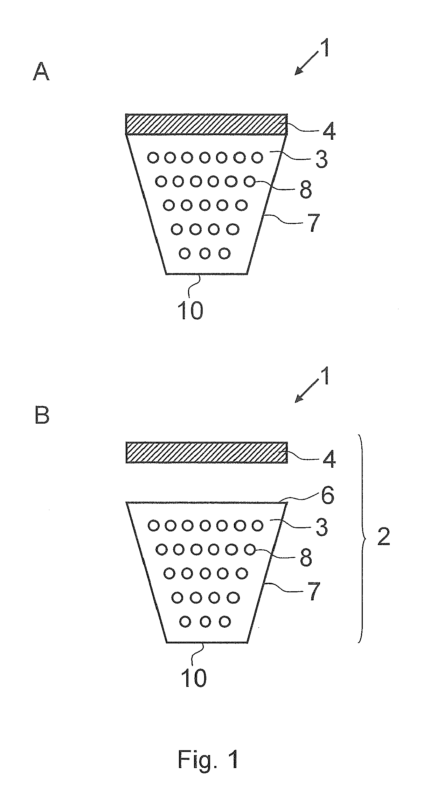

Figuren 1 A und B zeigen schematische Seitenansichten eines erfindungsgemässen Implantats 1 und seinen mehrteiligen Aufbau 2. Der mehrteilige Aufbau 2 umfasst einen insbesondere offenporigen Schaum 3 und eine insbesondere mikroporöse Membran 4. Die Membran 4 ist dem Schaum 3 an einem in bestimmungsgemässer Anwendungssituation nach aussen weisenden Ende 6 aufgelagert, wobei beide fest miteinander verbunden sind. An den Seiten 7 und dem gegenüberliegenden Ende 10 des Schaums 3 befinden sich keine Membranen. Die Membran 4 bildet eine planare 3-dimensionale Struktur ohne Aussparungen. Die Membran weist Poren auf (nicht gezeigt), welche die Membran 4 durchlässig für Flüssigkeiten und Dampf macht. Die Porengrösse ist so gewählt, dass die Membran undurchlässig für Mikroorganismen, insbesondere für Viren und/oder Bakterien, ist. Der Schaum 3 weist Poren 8 definierter Durchmesser auf, vorzugsweise mit Durchmessern von 40 bis 800 µm im trockenen Zustand. An den Seiten 7 und dem gegenüberliegendem Ende 10 des Schaums 3 sind die Poren zur Oberfläche hin offen. Bei einer bevorzugten Ausführungsform bilden die Poren durchgängige Kanäle aus, die ein gleichmässiges Vollsaugen des Implantats vermitteln. Erfindungsgemäss müssen der Schaum 3 und die Membran 4 nicht deckungsgleich sein. Bevorzugt sind der Schaum 3 und die

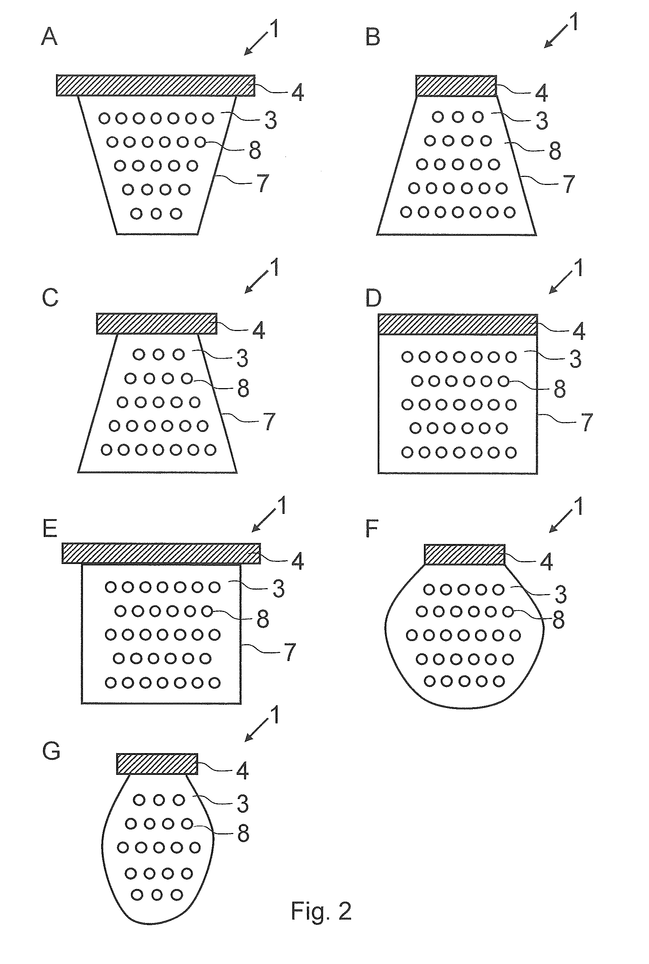

Membran 4 deckungsgleich. Die Membran 4 und der Schaum 3 können einander auch überlappen, insbesondere kann die Membran 4 den Schaum 3 an einer oder an mehreren Seiten überlappen. Das Implantat 1 kann beliebig ausgestaltet sein, beispielsweise kegelförmig, quaderförmig, zylinderförmig oder einer Zahnwurzel nachgeformt und/oder abgerundete Kanten aufweisen (vgl. Fig. 2). Zur Herstellung des Implantats sind vorteilhafte synthetische Zusammensetzungen im Obigen, insbesondere auf Basis von Polyurethan, beschrieben.

Figur 2 zeigt schematische Seitenansichten erfindungsgemässer Ausführungsformen des Implantats 1 mit Elementen des Implantats 1 aus Figur 1. Implantate 1 mit einer den Schaum 3 überlappenden Membran 4 sind gezeigt (Fig. 2 A, C, E, G). Ein zylinderförmiges Implantat 1 mit einem sich nach unten verjüngendem Schaum 3 kann erfindungsgemäss in einen Alveolarraum eingebracht werden, hierbei überlappt die Membran 4 den Schaum 3 (Fig. 2 A). In weiteren Ausführungsformen zylinderförmiger Implantate 1 ist die Membran 4 dem dünnen Ende des Schaums 3 aufgelagert und kann deckungsgleich oder überlappend ausgestaltet sein (Fig. 2 B, C). Quaderförmige oder kegelförmige Implantate bevorzugter Ausführungsformen weisen deckungsgleiche oder überlappende Kombinationen von Schaum 3 und Membran 4 auf (Fig. 2 D, E). In weiteren erfindungsgemässen Ausführungsformen des Implantats 1 ist der Schaum 3 kugelförmig ausgestaltet, wobei der Schaum 3 eine abgeflachte Seite im Kontaktbereich zur Membran 4 zeigt (Fig. 2 F). Das Implantat kann frei wählbare geometrische Formen, beispielsweise mit abgerundeten Kanten (Fig. 2 G), aufweisen.

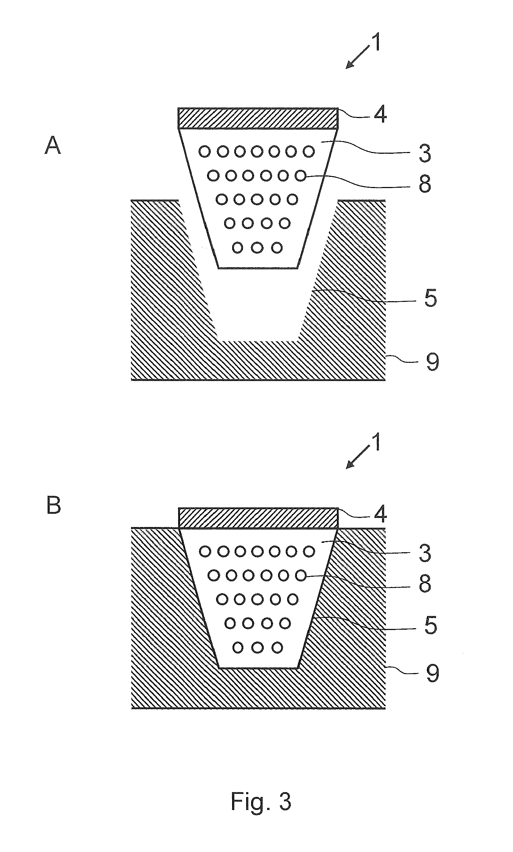

Figur 3 zeigt eine schematische Seitenansicht eines erfindungsgemässen Implantats 1 (vgl. Figur 1) und einen Knochenhohlraum 5. In bevorzugten Ausführungsformen ist das Implantat 1 derart dimensioniert, dass es in seinen Abmessungen dem Knochenhohlraum 5, beispielsweise einer Defektstelle nach einer Zahnextraktion, entspricht. Das Implantat 1 ist vor einem Einbringen in einen Knochenhohlraum 5 mit einer Schere und/oder einem Skalpell auf die Dimensionen des Knochenhohlraums 5 anpassbar. Dabei können die Abmessungen des Implantats kleiner, bevorzugt gleich oder grösser im Vergleich zu den Abmessungen der Defektstelle sein. Infolge der Volumen- und Formstabilität sowie der wählbaren Quelleigenschaften wird das Implantat nicht im Übermass wie beispielsweise Kollagenprodukte in den Defekt gestopft werden. Dadurch lassen sich die Porenstruktur und die Porengrössen im Defekt aufrechterhalten. Das Implantat 1 wird zur Verwendung in einem Knochenhohlraum 5, insbesondere einem Alveolarraum, zusammengedrückt und in den Knochenhohlraum 5 eingeführt. Mit einer Erholungszeit von insbesondere 2 bis 120 sec, bevorzugt 3-60 sec, nimmt das Implantat 1 sein ursprüngliches Volumen ein und passt sich dem Knochenholraum 5 an. Durch das Vollsaugen mit Gewebeflüssigkeit und besonders bevorzugt Blut kann das Implantat je nach Ausführungsform ein gewisses Quellvolumen aufweisen und/oder einen Quelldruck generieren. Es wird erwartet, dass ein konstanter Quelldruck im Bereich von mindestens 0,0001 N/mm² auf den Knochen 9 des Knochenhohlraums 5 einen volumenstabilisierenden Effekt ausübt und so während der Defektheilung einer Resorption der anliegenden Kieferknochenverhältnisse in horizontaler und vertikaler Richtung entgegen wirkt. Zugleich fixiert sich das Implantat auf diese Weise selbständig im Knochendefekt und muss nicht zwingend zusätzlich stabilisiert werden.
Aufgrund von Aufnahme von Feuchtigkeit, Gewebeflüssigkeit und/oder Blut quillt das Implantat 1 und übt einen Quelldruck von mindestens 0,0001 N/mm2 auf den umliegenden Knochen 9 aus. Weiter sorgen die auf das umliegende Gewebe wirkende Kräfte für ein vollständiges Ausfüllen des Knochenhohlraums 5 und damit für eine Volumenkonstanz während der *Guided Tissue Regeneration.* Der vorgenannte Quelldruck sorgt für eine ausreichende Befestigung des Implantats in dem Knochenhohlraum 5. Es können auch zusätzliche Befestigungsmittel appliziert werden, insbesondere Klammern, Nähte, Pins oder Schrauben.

Besonders bevorzugt wird das Implantat im trockenen Zustand eingebracht, so dass kurzzeitig ein hoher Schwelldruck wirkt, welcher das Implantat an die Dimensionen der Defektstelle anpasst. Dies wird im Folgenden durch eine Flüssigkeitsaufnahme, im Speziellen durch den sich aufbauenden Quelldruck, unterstützt.

Hier und im Folgenden ist Schwelldruck als Druckfestigkeit bei 60% vertikaler Kompression definiert.

Insbesondere bei der Verwendung des Implantats 1 in einem Alveolarraum, vorzugsweise nach Extraktion eines Zahns, liefern die feuchten Umgebungsbedingungen und/oder eine Blutung der Defektstelle verursacht durch die Zahnextraktion ausreichend Feuchtigkeit für ein Quellen des Implantats 1 und ein vollständiges Ausfüllen des Alveolarraums. Dabei erfolgt eine Verteilung des Wundblutes innerhalb des Implantats 1, insbesondere vermittelt durch die Poren 8 des Schaums 3. In der Folge bildet sich ein Blutkoagulum innerhalb des Schaums, welches durch das Implantat stabilisiert und geschützt wird. Die Membran 4 trennt Hart- und Weichgewebe, insbesondere Zahnfleisch und Kieferknochen, sodass eine separierte Regeneration der unterschiedlichen Gewebe erfolgt. Damit wird ein Einwachsen von schnellwachsendem Weichgewebe in den Alveolarraum verhindert. Die insbesondere mikroporöse Membran 4 dient der Mundschleimhaut und dem Bindegewebe als Leitstruktur für einen direkten Wundverschluss und reduziert oder verhindert während des Wundverschlusses den Eintritt von Fremdkörpern und Pathogenen aus der Mundhöhle in die Defektstelle. Die Membran 4 trägt auch zur Hämostase bei und verhindert zusätzlich die Blutung aus dem Defekt in die Mundhöhle. Das Blutkoagulum, welches durch das Implantat 1 stabilisiert und geschützt wird, stellt Wachstumsfaktoren, Osteoblasten und weitere essentielle Faktoren für die Knochenregeneration zur Verfügung, was ein Einwachsen von neuem Knochenmaterial in den Alveolarraum fördert. Die Resorptionseigenschaften des Implantats 1 sorgen für einen körpereigenen und rückstandsfreien Abbau des Implantats 1, vorzugsweise bei gleichzeitiger Bildung von neuem Hartgewebe, beispielsweise im Alveolarraum. Der durch das Implantat verursachte Quelldruck wird dabei konstant aufrechterhalten und vermindert sich zeitlich entsprechend der Implantatresorption. Die Resorption des Implantats 1 kann dabei auf die zeitlichen Resorptionsbedürfnisse von Hart- und Weichgewebe angepasst werden.

Die Erfindung wird im Folgenden anhand von Ausführungsbeispielen näher erläutert:

### Quelldruckmessung:

Die Messungen der Quelldrücke wurden an zylinderförmigen Probenkörpern mit Durchmesser von 10 mm und Höhe von 10 mm (Dimensionen im trockenen Zustand) durchgeführt. Dazu wurden die Probenkörper in eine allseitig begrenzte zylindrische Messkammer mit den Dimensionen Durchmesser von 10 mm und Höhe von 10 mm gelegt. Messkammer und Stempel sind aus poliertem Aluminium gefertigt. Die Messungen wurden bei Standardbedingungen durchgeführt (23 °C Raumtemperatur, 50% relative Luftfeuchtigkeit, 1013 hPa Standardatmosphärendruck). Über den Messkammerboden wurden die Probenkörper mit 0,9% NaCl-Lösung, welche eine Temperatur von 37°C aufweist, gewässert. Die Temperatur der Messkammer wurde konstant bei 37 °C gehalten, indem die Messkammer mit der temperierten 0,9% NaCl-Lösung umgeben war. Probenkörper wurden für 60 min in der mit Flüssigkeit gefluteten Messkammer positioniert, um ein Equillibrium der Quellung zu erreichen. Nach 60 min wurde der Quelldruck als Druckspannung gemessen. Jeder Materialtyp wurde mit drei verschiedenen Proben gemessen und die Messwerte gemittelt.

### Quellvolumen:

Die Volumenänderungen der zylindrischen Probenkörper infolge Flüssigkeitsaufnahme (0,9% NaCl, welche eine Temperatur von 37°C aufweist, für 60min) wurden bestimmt und prozentual angegeben (100% für das Volumen eines Zylinders mit Durchmesser von 10 mm und Höhe von 10 mm im trockenen Zustand).

### Probenkörper:

Die Vergleichsmessungen wurden für erfindungsgemässe unterschiedliche PUR-Schäume (PUR 1, 2, 3, 4) mit durchschnittlichen Porengrössen von 300 µm und 4 verschiedene kommerziell erhältliche Kollagen-Implantate (RESORBA PARASORB Sombrero™, RESOR-BA PARASORB Cone™, BIOPAD Wundkollagen™, Promogran Prisma™) durchgeführt. Die Probenkörper wurden auf die Dimensionen Durchmesser von 10 mm und Höhe von 10 mm zugeschnitten. Die Produkte Promogran Prisma™ und BIOPAD Wundkollagen™ sind nicht explizit für zahnmedizinische Zwecke vorgesehen. Sie bestehen aber ebenfalls aus Kollagenen wie die beiden genannten RESORBA Produkte sowie das Promogran Prisma™. Aufgrund der Verwendung von Kollagen weisen die vorgenannten Produkte grundsätzlich die gleichen Strukturen auf. Direkt für die Anwendung in der Alveole sind die beiden RESORBA Produkte vorgesehen.

Die folgende Tabelle zeigt Vergleichsmessungen des Quelldrucks, der Druckfestigkeit und des Quellvolumens erfindungemässer Implantate (PUR 1 bis 4) gegenüber handelsüblichen Knochenimplantaten. Die erfindungsgemässen Implantate zeichnen sich durch vorteilhafte Eigenschaften wie deutlich verbessertem Quelldruck, Druckfestigkeit und Quellvolumen aus.

Die Zusammensetzung des PUR 3 umfasst 4,55 g PEG-400, 2,61 g PVP, 13,64 g PEG-3000, 2 g Rizinusöl, 0,28 g Coscat 83, 0,23 g Jeffcat DMDEE, 0,5 g Ca-Stearat, 0,2 g Türkischrotöl, 0,32 g Wasser und 21,1 g Desmodur E 305.

**Tabelle: Vergleiche erfindungsgemässer Implantate und Kollagen-Implantate**

| Probenkörper (Zylinder: Durchmesser = Höhe = 10 mm, trocken) | Druckfestigkeit in N/mm² (trocken, seitlich begrenzt, 60% vertikale Kompression | Quelldruck in N/mm² (0,9% NaCl, 37°C, konstantes Volumen) | Quellvolumen resp. Schrumpfung in % (0,9% NaCl, 37°C, 60 min) |
|---|---|---|---|
| | | | |
| PUR 1 | 0.011 | 0.0038 | 200 |
| PUR 2 | 0.062 | 0.0161 | 230 |
| PUR 3 | 0.089 | 0.0037 | 330 |
| PUR 4 | 0.042 | 0.0012 | 360 |
| Parasorb Sombrero | * | << 0.0001 | 110 |
| Parasorb Dentalkegel | * | < 0.0001 | 100 |
| Biopad Wundkollagen | * | < 0.0001 | 90 |
| Promogran Prisma | * | < 0.0001 | 88 |

Im trockenen Zustand weisen die Probenkörper aus Kollagenen (RESORBA PARASORB Sombrero™, RESORBA PARASORB Cone™, BIOPAD Wundkollagen™, Promogran Prisma™) keine vergleichbare Druckfestigkeit wie die Probenkörper der PUR Materialien auf. Nach einer Stauchung auf 60% des Ursprungsvolumens bleibt der gestauchte Zustand bestehen (vgl. obenstehende Tabelle *). Die Quelldrücke der Kollagenmaterialien sind kaum oder nicht messbar und im Vergleich zu den PUR Materialien sehr gering.

Die Volumenmessung in nassem Zustand ist bei den Kollagenprodukten sehr schwierig, da sich die galert-artigen Probenkörper bereits bei leichter Berührung verformen. Werden die Probenkörper aus dem Quellmedium genommen, verlieren sie stark an Volumen da sie das Quellmedium nur begrenzt in der Matrix zu halten vermögen. Daher wurden die Messungen der Quellvolumen wie vorhergehend beschrieben in der 0,9% NaCl-Lösung durchgeführt.

## Patentansprüche

1. Implantat (1) zur Anwendung in einem Knochenhohlraum (5), welches im Wesentlichen synthetisches Material umfasst, insbesondere aus einem oder mehreren Polyurethanen, herstellbar oder hergestellt ist und dabei einen mehrteiligen Aufbau (2) aufweist, umfassend einen insbesondere offenporigen Schaum (3) und eine insbesondere mikroporöse Membran (4), **dadurch gekennzeichnet, dass** das Implantat (1) resorbierbar ist, und
a. viskoelastische Eigenschaften aufweist, insbesondere mit einer Druckfestigkeit von 0,0005 bis 0,1 N/mm², und/oder
b. einen Quelldruck, bevorzugt einen Quelldruck von 0,0001 bis 0,05 N/mm², besonders bevorzugt einen Quelldruck von 0,0004 bis 0,02 N/mm², aufweist.

2. Implantat (1) nach Anspruch 1, **dadurch gekennzeichnet, dass** die viskoelastischen Eigenschaften in nassen Umgebungsbedingen eine Druckfestigkeit von 0,001 bis 0,1 N/mm² aufweisen.

3. Implantat (1) nach einem der Ansprüche 1 und 2, **dadurch gekennzeichnet, dass** die Membran (4) den Schaum (3) an einem in bestimmungsgemässer Anwendungssituation nach aussen weisenden Ende (6) bedeckt.

4. Implantat (1) nach einem der Ansprüche 1 bis 3, **dadurch gekennzeichnet, dass** das Implantat (1) steril ist und/oder steril verpackt ist.

5. Implantat nach einem der Ansprüche 1 bis 4, **dadurch gekennzeichnet, dass** das eine oder die mehreren Polyurethane des Schaums (3) und der Membran (4) herstellbar oder hergestellt sind aus einer Zusammensetzung umfassend
- mindestens eine Polyol-Komponente umfassend mindest eine Verbindung mit mindestens zwei, vorzugsweise zwei, drei oder vier Hydroxidgruppen;
- mindestens eine Polyisocyanat-Komponente oder mindestens ein Polyurethan-Präpolymer umfassend mindesten eine Verbindung mit mindestens zwei, vorzugsweise zwei, drei oder vier Isocynatgruppen; und
- einen Zusatz, welcher mindestens eine der folgenden Substanzen enthält: Polyvinylpyrrolidon, Polyvinylpolypyrrolidon, sulfonierte Copolyester, Polyvinylalkylether, ein Copolymer von Polyvinylpyrrolidon mit Vinylacetat, Vinylimidazol oder Vinylcaprolactam, ein Copolymer von Alkylvinylether mit einer Säure oder einem Anhydrit, insbesondere Maleinsäure oder Maleinanhydrit, sowie Polymere und Copolymere der Acrylsäure und deren Salze oder Mischungen davon.

6. Implantat (1) nach einem der Ansprüche 1 und 5 **dadurch gekennzeichnet, dass** die Zusammensetzung des einen oder der mehreren Polyurethane des Schaums (3) zusätzlich mindestens eine der folgenden Komponenten umfasst:
- mindestens ein porenformendes Agens, vorzugsweise umfassend ein zweiwertiges Fettsäuresalz, speziell in Form eines Pulvers;
- einen Porenstabilisator, vorzugsweise ein Türkischrotöl oder Polyethersiloxan;
- mindestens einen Katalysator;
- mindestens ein Polycaprolacton, vorzugsweise Poly(ε-caprolacton);
- mindestens ein Mono- oder Polysaccharid, vorzugsweise Stärke;
- ein Polymer oder Copolymer einer Acrylsäure und ihres Salzes.

7. Implantat (1) nach einem der Ansprüche 4 bis 6, **dadurch gekennzeichnet, dass** die Zusammensetzung des einen oder der mehreren Poylurethane des Schaums (3) und/oder der Membran (4) mindestens eine Polyol-Komponente in Mengen von 1 bis 96 Gew.-% aufweist, vorzugsweise in Mengen von 30 bis 60 Gew.-%, besonders bevorzugt in Mengen von 35 bis 50 Gew.-%.

8. Implantat (1) nach einem der Ansprüche 4 bis 7, **dadurch gekennzeichnet, dass** der Schaum (3) das mindestens eine Polyurethan-Präpolymer oder die mindestens eine Polyisocyanat-Komponente in Mengen von 4 bis 60 Gew.-%, vorzugsweise in Mengen von 40 bis 60 Gew.-%, besonders bevorzugt in Mengen von 45 bis 55 Gew.-%, aufweist.

9. Implantat (1) nach einem der Ansprüche 4 bis 8, **dadurch gekennzeichnet, dass** der Zusatz in Mengen von 0,001 bis 10 Gew.-%, vorzugsweise zwischen 1 und 4 Gew.-%, in der Zusammensetzung des Schaums (3) und/oder der Membran (4) eingesetzt wird.

10. Implantat (1) nach einem der Ansprüche 4 bis 9, **dadurch gekennzeichnet, dass** das Polyvinylpyrrolidon der Zusammensetzung des einen oder der mehreren Polyurethane des Schaums (3) und/oder der Membran (4) ein Molekulargewicht zwischen 7'000 und 1'000'000 g/mol, vorzugsweise zwischen 40'000 und 60'000 g/mol, aufweist.

11. Implantat (1) nach einem der Ansprüche 4 bis 10, **dadurch gekennzeichnet, dass** die mindestens eine Polyol-Komponente des einen oder der mehreren Polyurethane des Schaums (3) und/oder der Membran (4) mindestens ein Polyethylenglycol umfassen.

12. Implantat (1) nach einem der Ansprüche 4 bis 11, **dadurch gekennzeichnet, dass** die mindestens eine Polyol-Komponente des einen oder der mehreren Polyurethane des Schaums (3) und/oder der Membran (4) mindestens ein Polycaprolacton, vorzugsweise ein Poly(ε-caprolacton), aufweist.

13. Implantat (1) nach einem der Ansprüche 1 bis 12, **dadurch gekennzeichnet, dass** der Schaum (3) Poren (8) mit einem Durchmesser zwischen 50 und 1000 µm im nassen Zustand aufweist.

14. Implantat (1) nach einem der Ansprüche 1 bis 13, **dadurch gekennzeichnet, dass** der Schaum (3) eine biologisch aktive Verbindung, vorzugsweise bereitgestellt in kristalliner Form, aufweist.

15. Implantat (1) nach einem der Ansprüche 1 bis 14, **dadurch gekennzeichnet, dass** der Schaum (3) ein Muster aufweist, welches das Schneiden des Schaums (3) auf spezifische Abmessungen und Formen ermöglicht.

16. Implantat (1) nach einem der Ansprüche 1 bis 15, **dadurch gekennzeichnet, dass** der Schaum (3) herstellbar oder hergestellt ist
- mittels eines insbesondere kontinuierlichen Sprühauftragsverfahrens, oder
- in einem Formwerkzeug geschäumt, oder
- durch Gefriertrocknung einer Suspension.

17. Implantat (1) nach einem der Ansprüche 1 bis 16, **dadurch gekennzeichnet, dass** die Membran (4) eine Dicke von 10 bis 1'500 µm aufweist, vorzugsweise 20 bis 500 µm, besonders bevorzugt 50 bis 300 µm.

18. Implantat (1) nach einem der Ansprüche 4 bis 17, **dadurch gekennzeichnet, dass** die Membran (4) das mindestens eine Polyurethan-Präpolymer oder die mindestens eine Polyisocyanat-Komponente in Mengen von 4 bis 60 Gew.-%, vorzugsweise in Mengen von 40 bis 60 Gew.-%, besonders bevorzugt in Mengen von 45 bis 55 Gew.-%, aufweist.

19. Ein System umfassend Implantate (1), insbesondere gemäss einem der Ansprüche 1 bis 18, mit unterschiedlichen Abmessungen und Volumen, vorzugsweise im Bereich von 0,5 bis 30 mm respektive von 0,125 bis 27'000 mm³, und/oder unterschiedlichen Porengrössen des Schaums (3) mit Instruktionen zur Anwendung in einem Knochenhohlraum.
